(19) **Europäisches Patentamt
European Patent Office
Office européen des brevets**

(11) **EP 1 573 334 B1**

(12) **FASCICULE DE BREVET EUROPEEN**

(45) Date de publication et mention
de la délivrance du brevet:
**25.07.2007 Bulletin 2007/30**

(51) Int Cl.:
**G01N 33/574** (2006.01)    **G01N 33/68** (2006.01)

(21) Numéro de dépôt: **03799634.5**

(22) Date de dépôt: **18.12.2003**

(86) Numéro de dépôt international:
**PCT/FR2003/003802**

(87) Numéro de publication internationale:
**WO 2004/057337 (08.07.2004 Gazette 2004/28)**

(54) **METHODE D'ANALYSE DE L'AGRESSIVITE TUMORALE COMPRENANT LA MESURE D'ACTINE POLYMERISEE**

VERFAHREN ZUM UNTERSUCHEN VON KREBS AGGRESSIVITÄT MIT POLYMERISIERTE AKTIN BESTIMMUNG

METHOD FOR ANALYZING TUMOR AGRESSIVITY COMPRISING MEASUREMENT OF POLYMERIZED ACTIN

(84) Etats contractants désignés:
**AT BE BG CH CY CZ DE DK EE ES FI FR GB GR HU IE IT LI LU MC NL PT RO SE SI SK TR**

(30) Priorité: **19.12.2002 FR 0216188**

(43) Date de publication de la demande:
**14.09.2005 Bulletin 2005/37**

(73) Titulaires:
• **Bioalliance Pharma**
**75015 Paris (FR)**
• **ECOLE NORMALE SUPERIEURE DE CACHAN**
**94235 Cachan (FR)**
• **Centre National De La Recherche Scientifique-CNRS**
**75794 Paris Cedex 16 (FR)**

(72) Inventeurs:
• **PRAGNON, Christelle**
**F-37510 Savonnière (FR)**
• **POLARD, Valérie**
**F-94140 Alfortville (FR)**
• **SUBRA, Frédéric**
**F-75009 Paris (FR)**
• **AUCLAIR, Christian**
**F-75011 Paris (FR)**

(74) Mandataire: **Breese, Pierre**
**BREESE DERAMBURE MAJEROWICZ**
**38, avenue de l'Opéra**
**75002 Paris (FR)**

(56) Documents cités:
WO-A-01/41815        WO-A-01/71356
WO-A-99/11814        US-A- 5 851 786

EP 1 573 334 B1

## Description

La présente invention a pour objet un test diagnostic ou prédictif dans le domaine du cancer. Plus particulièrement, l'invention se rapporte à une méthode basée sur la mesure directe de la quantité d'actine polymérisée à l'état stationnaire dans un lysat cellulaire non purifié, comme indicateur à la fois de l'agressivité tumorale et de la sensibilité au traitement anti-tumoral.

Selon une forme de mise en oeuvre préférée, la a méthode de l'invention est remarquable en ce que, à part l'extrait cellulaire et l'actine fluorescente, aucun ajout supplémentaire protéique purifié n'est nécessaire pour mettre en évidence une discrimination à la fois entre des cellules sensibles ou résistantes et des cellules à potentiel agressif ou non.

Le cytosquelette d'actine est une structure protéique essentielle à la survie cellulaire. Il permet notamment à la cellule de maintenir sa forme et son adhésion, de migrer, de communiquer avec les cellules adjacentes. Le cytosquelette est une structure extrêmement dynamique, en perpétuel remaniement.

Les propriétés dynamiques du cytosquelette permettent à la cellule de communiquer avec l'environnement extérieur et de migrer pour former des métastases par exemple. Ces deux propriétés sont à l'origine de la cohésion tissulaire.

Les propriétés dynamiques du cytosquelette rendent son étude extrêmement difficile.

Il a été décrit dans l'art antérieur plusieurs mécanismes de régulation de la polymérisation de l'actine, parmi lesquels on peut citer les mécanismes dépendant soit du complexe Arp2/3, soit faisant intervenir la famille de protéines reliées à Ena/VASP.

Ainsi, le complexe Arp2/3, constitué d'au moins sept sous-unités protéiques, est régulé par les membres de la famille de protéines du syndrome de Wiskott-Aldrich (WASP) pour activer la nucléation des filaments d'actine en structures en Y (Machesky, Mullins et al.1999).

Pour Ena/VASP, il s'agit de la famille des protéines dont le prototype est la zyxine, parmi lesquelles on peut citer LPP (LIM-containing lipoma preferred partner), et TRIP6 (thiroid receptor-interacing protein-6), caractérisées en ce qu'elles possèdent un domaine riche en proline suivi par trois domaines LIM. Les protéines appartenant à cette famille interagissent avec les protéines de la famille Ena/VASP parmi lesquelles on peut cite VASP (Vasodilator stimulated phosphoprotein), Ena (chez la drosophile), Mena (équivalent de la protéine Ena chez les mammifères) et Ev1. Bien que ces deux dernières familles de protéines soient clairement impliquées dans la régulation de ce mécanisme de polymérisation de l'actine, la séquence d'événements responsables n'est pas complètement élucidée (Fradelizi, Noireaux et al. 2001).

Il semble qu'une même cellule puisse contenir plusieurs systèmes de polymérisation de l'actine, chacun étant responsable d'un type particulier de structure d'actine.

Les propriétés dynamiques du cytosquelette rendent son analyse extrêmement complexe, compte tenu des systèmes protéiques différents intervenant dans les processus de régulation de la polymérisation et de la dépolymérisation de l'actine. Si les propriétés d'adhésion et de résistance sont liées (Damiano, Hazlehurst et al. 2001 ; dit Faute, Laurend et al. 2002), les propriétés invasives des cellules cancéreuses diffèrent selon leur résistance aux traitements anti-cancéreux (Lopes, Ernst et al. 2002). In vivo, les cellules résistantes semblent avoir un pouvoir invasif plus grand (Mitsumoto, Kamura et al. 1998).

Ainsi, l'étude d'un seul des deux mécanismes dans un système purifié *in vitro* peut être incomplet pour juger du potentiel cellulaire de polymérisation de l'actine d'un extrait d'une lignée cellulaire ou d'un prélèvement biologique.

Les travaux de recherche réalisés dans le cadre de la présente invention ont maintenant mis en évidence une nette corrélation entre d'une part la mesure de la quantité d'actine polymérisée à l'état stationnaire, et d'autre part l'agressivité tumorale.

La présente invention a donc pour but d'offrir une méthode simple et efficace de diagnostic ou de prédiction de l'agressivité tumorale et de la sensibilité au traitement anti-cancéreux chez un sujet. Ce but est atteint grâce à une méthode consistant à mesurer la quantité d'actine polymérisée à l'état stationnaire dans un extrait cellulaire d'un prélèvement d'un sujet.

Plus particulièrement, l'invention a pour objet une méthode d'analyse de l'agressivité tumorale de cellules cancéreuses chez un sujet comprenant la mesure de la quantité d'actine polymérisée à l'état stationnaire dans un extrait cellulaire du sujet.

Avantageusement, l'extrait cellulaire est un lysat desdites cellules cancéreuses.

On entend par agressivité tumorale le caractère invasif d'un cancer ou d'une lignée cellulaire cancéreuse, c'est-à-dire aussi bien le potentiel métastatique de ceux-ci que la rapidité avec laquelle une tumeur primitive se développe et croît. On entend également par agressivité tumorale, la tumorigénicité, c'est-à-dire la capacité d'une lignée cellulaire à entraîner plus ou moins efficacement l'apparition d'une tumeur après injection en sous cutanée dans un modèle murin apte à recevoir cette lignée. On entend aussi par agressivité tumorale l'absence de sensibilité au traitement anti-cancéreux.

La mesure réalisée sur l'extrait cellulaire du sujet est comparée à une ou plusieurs valeurs de référence spécifiques du tissu analysé dans le cas de prélèvements biologiques, ou spécifiques du phénotype, dans le cas de lignées cellulaires.

La quantité d'actine polymérisée correspond à la somme de toute l'actine sous forme F. Cette somme dépend de la quantité totale d'actine, quelle que soit sa forme, forme F ou forme G, mais surtout de l'ensemble des mécanismes de régulation de la polymérisation et de la dépolymérisation.

On entend par actine F les polymères plus ou moins longs d'actine globulaire (actine G). La formation d'actine F est un phénomène dynamique, très précisément régulé par plusieurs mécanismes différents. L'actine F peut subir de manière concomitante un phénomène de polymérisation d'actine globulaire à une extrémité du filament et un phénomène de dépolymérisation à l'autre extrémité.

Un but de la présente invention est de s'affranchir des différentes voies de régulation de la polymérisation de l'actine, en utilisant un système intégré.

Ce but est atteint selon l'invention, grâce à la mesure de la quantité d'actine polymérisée à l'état stationnaire, laquelle prend en compte la résultante de tous les mécanismes de régulation de la polymérisation et de la dépolymérisation des filaments d'actine, et plus précisément, aussi bien les mécanismes de stimulation que les mécanismes d'inhibition de la polymérisation et de la dépolymérisation. L'état stationnaire résulte de l'équilibre entre tous ces mécanismes de régulation de la polymérisation de l'actine à une extrémité du filament d'actine et de la dépolymérisation à l'autre extrémité.

De façon avantageuse, la mesure de l'actine polymérisée à l'état stationnaire ne nécessite pas une mesure d'expression d'une protéine (par exemple la zyxine ou l'une des protéines du complexe Arp2/3 ou l'une des protéines de la famille Ena/VASP) et ne nécessite pas d'étape de purification protéique ou l'ajout de réactifs solides (par exemple des billes).

La présente invention permet de mesurer la quantité d'actine polymérisée à l'état stationnaire d'un extrait cellulaire non purifié, où toutes les voies d'activation/inhibition des processus de polymérisation et dépolymérisation de l'actine sont intégrées.

Ainsi, on entend par quantité d'actine polymérisée à l'état stationnaire, la quantité d'actine polymérisée lorsque l'équilibre entre la polymérisation de l'actine à une extrémité des filaments et la dépolymérisation à l'autre extrémité est atteint. Comme indiqué précédemment, l'état stationnaire est la résultante de toutes les voies de régulation de la polymérisation de l'actine.

La mesure de la quantité d'actine à l'état stationnaire peut être réalisée par toute technique connue de l'homme du métier, comme par exemple la technique de polarisation statique de fluorescence aussi appelée anisotropie statique de fluorescence.

L'anisotropie et la polarisation sont deux valeurs reliées mathématiquement et donc facilement interchangeables. Elles décrivent le même phénomène. La polarisation de fluorescence permet d'étudier les interactions entre molécules en mesurant les changements de taille de molécules fluorescentes en solution. Cette mesure est corrélée à la taille de la molécule fluorescente ou du complexe moléculaire fluorescent. En l'occurrence, la molécule fluorescente est un monomère d'actine (ou actine G) lié à un fluorochrome, en l'occurrence l'Alexa 488, qui est incorporé dans les filaments d'actine (actine F) au cours de la polymérisation.

Ainsi, selon une forme toute préférée de réalisation, la mesure de la quantité d'actine à l'état stationnaire de la méthode d'analyse de l'invention est réalisée par polarisation statique de fluorescence en présence de monomères d'actine liés à un fluorochrome, lesquels sont incorporés dans les filaments d'actine (actine F) formés au cours de la polymérisation de l'actine endogène du lysat.

Dans cette forme de réalisation, les monomères d'actine liés à un fluorochrome sont ajoutés au lysat cellulaire dans un rapport compris entre 1/80e et 1/1600e par rapport à la quantité d'actine endogène.

Le résultat du test est une valeur d'anisotropie de fluorescence au plateau (« STAFI ») correspondant à la quantité d'actine polymérisée à l'état stationnaire et une constante apparente ($K_{obs}$) de polymérisation de l'actine résultant de l'incorporation progressive des monomères d'actine marquées au cours du temps jusqu'à atteindre l'état stationnaire, c'est-à-dire le plateau de la courbe.

Grâce au logiciel GraphPad Prism® version 3 (GraphPad Software Inc.), les données expérimentales permettent de générer une courbe ajustée à une équation d'ordre 1 telle que :

$$Y = \Delta mA\ max\ (1 - e^{-K.t})$$

où

Y = la valeur d'anisotropie mesurée à un temps t
$\Delta$mA max = « STAFI », la valeur d'ordonnée maximum à l'équilibre
K = la constante $K_{obs}$
t = le temps-en secondes

Pour prédire un niveau d'agressivité tumorale et de sensibilité à un traitement anti-cancéreux, ces deux valeurs doivent être comparées à des valeurs de référence soit spécifiques du tissu analysé dans le cas de prélèvements biologiques, soit spécifiques du phénotype, dans le cas de lignées cellulaires.

Un échantillon provenant d'un prélèvement de cancer très agressif, c'est-à-dire présentant soit un caractère invasif soit un caractère tumorigène ou bien encore ayant perdu le caractère de sensibilité aux traitements anti-cancéreux, présentera un STAFI et un $K_{obs}$ plus faibles que la valeur de référence, c'est-à-dire la valeur obtenue à partir de prélèvements similaires peu ou pas agressifs.

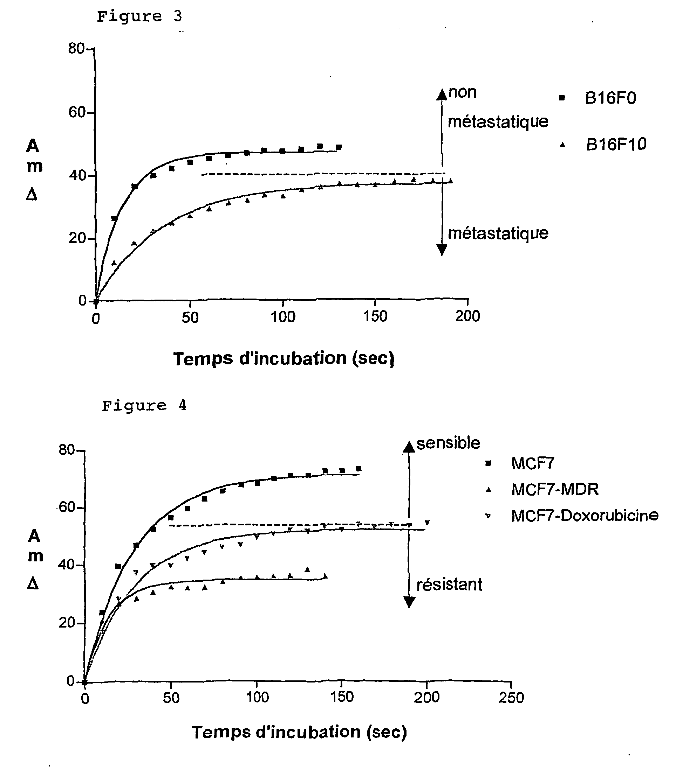

Par exemple, la valeur normale d'anisotropie de fluorescence au plateau (« STAFI » = delta mA max) d'une lignée peu invasive de mélanome (B16F0) est 47mA ($K_{obs}$ = 0,07). Une lignée dérivée de celle-ci, décrite par ailleurs comme très invasive (B16F10) (Nakamura, Yoshikawa et al. 2002), présente une valeur de STAFI de 37 mA ($K_{obs}$ = 0,02), nettement inférieure à la valeur des B16F0. Les valeurs de STAFI et de Kobs obtenues à partir de lysats de cellules invasives sont nettement inférieures aux valeurs de la lignée de référence (figure 3).

Un autre exemple est la comparaison de la valeur du STAFI de lignées tumorigènes avec la valeur obtenue à partir de lignées cellulaires parentales non tumorigènes prises comme référence. La valeur du STAFI des lignées tumorigènes est nettement inférieure aux valeurs des lignées de référence non tumorigènes.

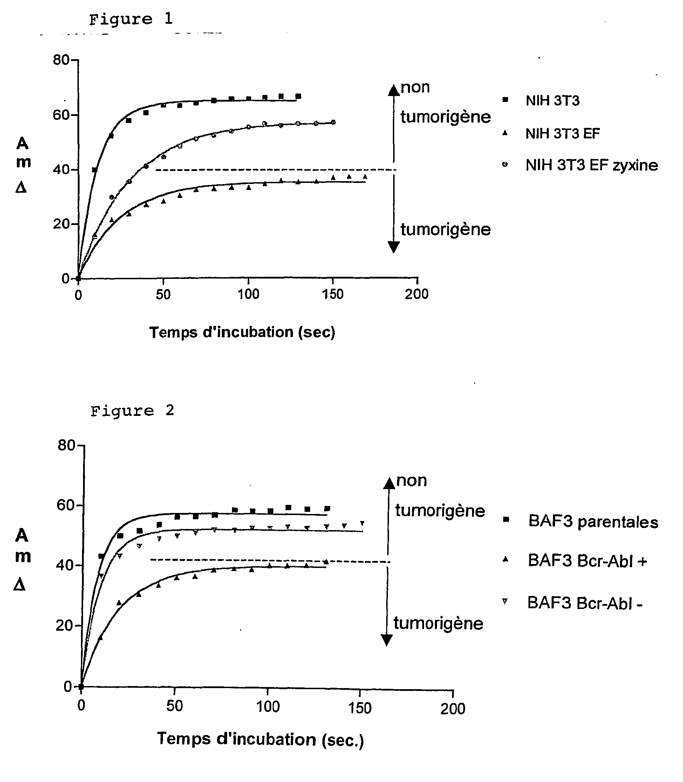

Dans l'exemple des lignées dérivées de NIH 3T3, le STAFI de la lignée tumorigène (NIH 3T3 EF) est égal à 35mA comparé aux lignées de référence non tumorigènes (NIH 3T3 et NIH 3T3 EF zyxine) pour lesquelles le STAFI est égal à 65 et 57 mA respectivement (figure 1).

La lignée tumorigène BAF3 bcr-abl et la lignée non tumorigène BAF3 présentent des valeurs de STAFI de 40mA et 58mA respectivement. La répression de l'expression de l'oncogène de fusion, responsable de la tumorigénicité de la lignée BAF3 bcr-abl, induit la restauration de la valeur du STAFI à une valeur proche du STAFI de la lignée de référence soit 52mA et 58mA respectivement (figure 2).

Un dernier exemple est la comparaison de la valeur de STAFI de lignées cellulaires de cancer du sein plus ou moins sensibles au traitement anticancéreux. Les valeurs de STAFI des deux lignées résistantes (MCF7-MDR et MCF7-dox) sont nettement inférieures à la valeur de la lignée sensible (MCF7) prise comme référence soit 35mA et 52mA versus 71mA (figure 4).

Un exemple préféré de mise en oeuvre de la méthode selon l'invention comprend les étapes suivantes :

- la lyse des cellules cancéreuses dans des conditions non-dénaturantes pour les protéines et l'élimination des débris cellulaires,
- le dosage des protéines totales du lysat,
- l'ajout de monomères d'actine liés à un fluorochrome,
- l'ajout des substances nécessaires à la polymérisation de l'actine endogène et à la protection des protéines du lysat,
- la mesure de la quantité d'actine polymérisée à l'état stationnaire dans le lysat.

La présente invention vise aussi à offrir une méthode d'identification de molécules susceptibles de présenter une activité anticancéreuse. Un telle méthode comprend la mise en oeuvre de la méthode d'analyse de l'agressivité tumorale décrite précédemment selon l'une quelconque des revendications 1 à 6 en présence d'une quantité adéquate d'une ou plusieurs molécules à tester, et la détermination de la capacité de ladite molécule à restaurer une quantité d'actine polymérisée à l'état stationnaire correspondant à celle de cellules non agressives.

Les travaux réalisés dans le cadre de la présente invention ont permis l'identification de molécules capables de restaurer la valeur du STAFI de cellules agressives au niveau de celle de cellules non agressives. Ces molécules sont susceptibles de présenter une activité anti-cancéreuse.

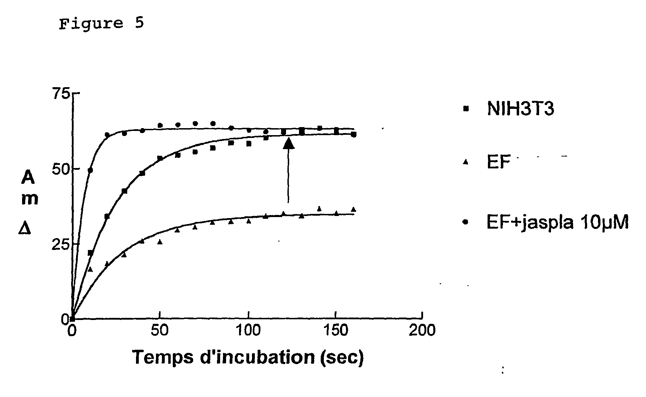

Par exemple, la Jasplakinolide, ajoutée dans le lysat de cellules agressives (NIH 3T3 EF) juste au moment du test, permet la restauration de la valeur du STAFI de ces cellules à une valeur proche de celle du STAFI de cellules de référence non tumorigènes (NIH 3T3) (figure 5). Le tableau 1 ci-dessous rapporte la restauration de la valeur de delta mA max (STAFI, correspondant à la quantité d'actine polymérisée à l'état stationnaire) de cellules tumorigènes (NIH 3T3 EF, notées EF) au niveau de la valeur de cellules non tumorigènes (NIH 3T3)par l'ajout de la jasplakinolide (notée jaspla).

Tableau 1

| Lignées cellulaires | $\Delta$mA max |
|---|---|
| NIH 3T3 EF | 30 |
| NIH 3T3 | 62 |
| NIH 3T3 EF + Jasplakinolide | 65 |

L'invention concerne encore l'application de la méthode d'analyse de l'agressivité tumorale décrite précédemment à :

- l'évaluation du caractère invasif desdites cellules ;
- l'évaluation de la tumorigénicité des cellules ;
- la prédiction de la sensibilité desdites cellules à un traitement anti-cancéreux ; le traitement anti-cancéreux consiste par exemple en une radiothérapie ou une chimiothérapie.

On entend par sensibilité au traitement anti-cancéreux aussi bien l'absence de résistance aux médicaments dépendant du système MDR (multi drug resistance) liée aux mécanismes de pompes de la famille de protéines P-gp, que la capacité des cellules cancéreuses à entrer en apoptose. Ces deux phénomènes peuvent être en réponse à un traitement anti-cancéreux consistant en une radiothérapie ou une chimiothérapie.

L'invention vise également un kit pour un test diagnostic ou prédictif de l'agressivité tumorale, et plus spécialement pour la mesure de la quantité d'actine polymérisée à l'état stationnaire pour l'évaluation de l'agressivité tumorale dans un prélèvement biologique.

Un tel kit comprend :

- un milieu de re-suspension des cellules pour la lyse des cellules,
- les substances nécessaires à la polymérisation de l'actine endogène et à la protection des protéines du lysat,
- les monomères d'-actine liés à un fluorochrome,
- un tampon de polymérisation de l'actine,
- un tampon général de l'actine,
- éventuellement des extraits de référence de cellules agressives et non agressives.

D'autres avantages et caractéristiques de l'invention apparaîtront des exemples qui suivent dans lesquels il sera fait référence aux dessins en annexe où :

- La figure 1 illustre la mesure de la polymérisation de l'actine à l'état stationnaire (STAFI = delta mA max) dans des lignées cellulaires parentales adhérentes murines, non tumorigènes (NIH 3T3 et NIH 3T3 EF zyxine) et tumorigène (NIH 3T3 EF).
- La figure 2 illustre la mesure de la polymérisation de l'actine à l'état stationnaire (STAFI = delta mA max) dans des lignées cellulaires parentales non adhérentes (cellules hématopoïétiques murines), non tumorales (BAF3 et BAF3 Bcr-Abl⁻) et tumorale (BAF3 Bcr-Abl⁺) .
- La figure 3 illustre la mesure de la polymérisation de l'actine à l'état stationnaire (STAFI = delta mA max) dans des lignées cellulaires parentales de carcinome du sein, sensible (MCF7) et résistantes (MCF7-Doxorubicine et MCF7-MDR).
- La figure 4 illustre la mesure de la polymérisation de l'actine à l'état stationnaire (STAFI = delta mA max) dans des lignées cellulaires parentales de mélanome, ayant un potentiel plus ou moins métastatique (B16F0 < B16F10).
- La figure 5 illustre l'identification d'une molécule (la jasplakinolide) capable de restaurer la quantité d'actine à l'état stationnaire (STAFI = delta mA max) d'une lignée tumorigène au niveau de la quantité d'une lignée non tumorigène.

I - Méthodes.

La technique de polarisation de fluorescence aussi appelée anisotropie statique de fluorescence permet d'obtenir une valeur dépendant de la taille de la molécule fluorescente et du nombre de complexes moléculaires fluorescents. C'est-à-dire qu'en ajoutant une faible proportion d'actine monomèrique fluorescente dans un extrait cellulaire, ces monomères vont être incorporés dans le filament d'actine au cours de la polymérisation ce qui va entraîner une augmentation de la valeur d'anisotropie jusqu'à un plateau apparent, correspondant à l'équilibre entre la polymérisation et la dépolymérisation des filaments d'actine contenant des monomères fluorescents. Le niveau de ce plateau, appelé « STAFI », reflète la quantité d'actine F à l'état stationnaire, c'est-à-dire le potentiel adhésif de la cellule, et la vitesse d'obtention du plateau dépend de la rapidité avec laquelle ces filaments se forment. Ces deux paramètres sont des index de l'agressivité tumorale.

Méthode de lyse des cellules : Pour les cellules en culture, les cellules peuvent devoir être trypsinées avant d'être lavées (tampon de lavage : 135 mM NaCl, 2.7 mM KCl, 11.9 mM NaHCO$_3$, 0.36 mM NaH$_2$PO$_4$, 2 mM MgCl$_2$, 0.2 mM EGTA, 5.5 mM glucose, 0.3 % albumine, pH=6,5). Les cellules sont mises en suspension dans un tampon de sonication (10 mM Tris-HCl, pH 7,5, 10 mM EGTA et 2 mM MgCl$_2$ + inhibiteurs de protéases Roche) à raison de 50.10$^6$ cellules/ml puis soniquées sur glace, à raison de 10 runs de 10 secondes espacés de temps de pause de 30 secondes. Le lysat est centrifugé 30 min à 8000 rpm à 4 °C puis filtré sur 0.45 $\mu$m. La concentration en protéines totales du lysat est mesurée par la méthode de Bradford pour être ajustée à 2mg/ml avec du tampon de sonication. 0,4mM final d'ATP et de DTT sont ajoutés pour constituer le lysat cellulaire à tester.

La solution de monomères d'actine liés à un fluorochrome est préparée de la manière suivante : la solution stock d'actine-Alexa 488 (Molecular Probes) (7,3 mg/ml) est diluée au 1:200e dans du tampon G (5 mM Tris pH8,1, 0,2 mM CaCl$_2$, 0,2 mM DTT, 0,2 mM ATP) supplémenté par 10% de sucrose puis ultracentrifugée à 35 000 rpm, 120 min, à 4°C afin d'éliminer d'éventuels filaments d'actine. Cette solution de monomères d'actine marqués est conservée à - 80°C en aliquots.

Au moment du test, la solution de monomères d'actine marqués est diluée au 1/3 dans du tampon G. L'appareil utilisé est un Beacon 2000. Introduire dans un tube pour Beacon, 167 $\mu$l de tampon G et 3 $\mu$l de solution de monomères d'actine marqués préalablement dilué. Après stabilisation de la valeur d'anisotropie des monomères d'actine à environ 110 mA , ajouter 4 $\mu$l de tampon de polymérisation (2,5 M KCl, 50 mM MgCl$_2$, 25 mM ATP) et 20 $\mu$l d'extrait cellulaire à tester à 2 mg/ml. La valeur d'anisotropie de fluorescence est enregistrée pendant une période d'environ 200 secondes. Les données sont traitées avec le logiciel GraphPad Prism version 3.0 (Ed. GraphPad Software). La valeur d'anisotropie de fluorescence des monomères d'actine marqués seuls (environ 110mA) est retranchée des valeurs suivantes.

L'ensemble des lignées cellulaires est cultivé à 37°C en atmosphère humide contenant 5% de CO$_2$. Elles sont entretenues dans du milieu DMEM ou RPMI (Gibco) supplémenté par 10% de sérum de veau nouveau-né ou de sérum de veau foetal (Gibco) et d'antibiotiques (pénicilline à 100UI/mL et streptomycine à 100$\mu$g/mL).

La lignée NIH-3T3 est une ligné de fibroblastes murins non tumorigènes.

La lignée NIH-3T3-EF est une lignée tumorale dérivée de la précédente et contient un ADNc codant pour l'oncogène de fusion EWS-FLI dans son génome. L'expression de cette protéine est sélectionnée à l'aide de 2,5$\mu$g/mL de puromycine.

La lignée NIH-3T3-EF-zyxine est une lignée dérivée de la précédente, qui a perdu son caractère tumorigène suite à la transformation par un ADNc codant pour la protéine zyxine humaine. L'expression de cette protéine est sélectionnée à l'aide de la généticine.

La lignée BAF3 est une lignée pré-lymphocytaire murine. Elle est entretenue en présence d'IL3.

La lignée BAF3 Bcr-Abl est une lignée dérivée de la précédente, contenant un ADNc codant pour l'oncogène de fusion Bcr-Abl, dont l'expression peut être réprimée par la doxicycline. Lorsque cette lignée est cultivée en l'absence de doxicycline et d'IL3, l'oncogène est exprimé et elle est alors notée BAF3 bcr-abl$^+$. Lorsque cette lignée est cultivée en présence de doxicycline et d'IL3, l'oncogène de fusion n'est plus exprimée et elle est notée BAF3 bcr-abl$^-$(Dugray, Geay et al. 2001).

La lignée B16F0 est une lignée de mélanome murin ayant un faible potentiel métastatique (Nakamura, Yoshikawa et al. 2002).

La lignée B16F10 est une lignée dérivée de la précédente, ayant acquis un fort potentiel métastatique à la suite de 10 sélections successives dans un modèle murin syngénique de métastases pulmonaires (Nakamura, Yoshikawa et al. 2002).

La lignée MCF7 est une lignée de carcinome mammaire humain faiblement tumorigène.

La lignée MCF7-Dox est une lignée dérivée de la précédente, rendue résistante à la doxorubicine par ajout régulier de 10μm de doxorubicine dans le milieu de culture.

La lignée MCF7-MDR est une lignée dérivée de la lignée MCF7, contenant un ADNc codant pour la P-gp, responsable de la perte de la sensibilité à la chimiothérapie des cellules cancéreuses.

II - Résultats.

1) Corrélation entre la tumorigénicité de lignées cellulaires adhérentes de fibroblastes murins et les valeurs mesurées de STAFI et K$_{obs}$.

La figure 1 représente la détermination de la valeur de delta mA max (STAFI, correspondant à la quantité d'actine polymérisée à l'état stationnaire) dans trois lignées cellulaires parentales murines adhérentes, non tumorigènes (NIH 3T3 et NIH 3T3 EF zyxine) et tumorigènes (NIH 3T3 EF). Le tableau 2 ci-dessous rapporte la cinétique de polymérisation de l'actine en présence d'extraits cellulaires par polarisation statique de fluorescence.

Tableau 2

| Lignées cellulaires | Delta mA max | Kobs |
|---|---|---|
| NIH 3T3 | 65 | 0,086 |
| NIH 3T3 EF | 35 | 0,04 |
| NIH 3T3 zyxine | 57 | 0,033 |

La quantité d'actine polymérisée à l'état stationnaire (« STAFI » : valeur d'anisotropie de fluorescence au plateau) de la lignée tumorigène (NIH 3T3 EF) est comparée à celle de la lignée parentale non tumorigène (NIH 3T3) (figure 1). Le delta mA max (SATFI) de la lignée tumorigène (NIH 3T3 EF) est nettement inférieur au delta mA max de la lignée non tumorigène (NIH 3T3), soit 35 et 65 respectivement.

L'expression de la zyxine dans la lignée tumorigène NIH 3T3 EF conduit à une importante diminution de la tumorigénicité de cette lignée (NIH 3T3 EF Zyxine). La diminution de la tumorigénicité de cette lignée est corrélée avec la restauration du delta mA max à une valeur proche de celle de la lignée de référence, en l'occurrence la lignée NIH 3T3, soit 57 et 65 respectivement.

2) Corrélation entre la tumorigénicité de lignées cellulaires pré-lymphocytaires murines non adhérentes et les valeurs mesurées de STAFI et K$_{obs}$.

La figure 2 représente la détermination de la valeur de delta mA max (STAFI, correspondant à la quantité d'actine polymérisée à l'état stationnaire) dans différentes lignées cellulaires non adhérentes, non tumorigènes (BAF3 parentales, BAF3 Bcr-Abl$^-$) et tumorigène (BAF3 Bcr-Abl$^+$). Le tableau 3 rapporte la cinétique de polymérisation de l'actine en présence d'extraits cellulaires par polarisation statique de fluorescence.

Tableau 3

| Lignées cellulaires | Delta mA max | Kobs |
|---|---|---|
| BAF3 parentales | 58 | 0,12 |
| BAF3 Bcr-Abl$^+$ | 40 | 0,05 |
| Bcr-Abl$^-$ | 52 | 0,101 |

La quantité d'actine polymérisée à l'état stationnaire (« STAFI » : valeur d'anisotropie de fluorescence au plateau, c'est-à-dire la valeur de delta mA maximum) de la lignée tumorigène BAF3 Bcr-Abl$^+$, transformée par l'oncogène de fusion Bcr-Abl, est comparée à celle de la lignée parentale non tumorigène (BAF3) (figure 2) (Dugray, Geay et al. 2001). Pour la lignée tumorigène (BAF3 Bcr-Abl$^+$), le delta mA max est nettement inférieur au delta mA max de la lignée non tumorigène (BAF3), soit 40 et 58 respectivement.

La même observation est faite pour la vitesse de polymérisation de l'actine, représentée par la constante K$_{obs}$. Pour la lignée tumorigène (BAF3 Bcr-Abl$^+$), K$_{obs}$ est nettement inférieur au K$_{obs}$ de la lignée non tumorigène (BAF3), soit 0,05 et 0,12 respectivement.

La répression de l'expression de l'oncogène de fusion par la doxicycline dans la lignée BAF3 Bcr-Abl conduit à la perte de la tumorigénicité de cette lignée (Dugray, Geay et al. 2001). La répression de l'expression de l'oncogène restaure le delta mA max de cette lignée tumorigène à une valeur proche de celle de la lignée non tumorigène de référence, soit 52 et 58 respectivement.

3) Corrélation entre la sensibilité à un traitement anti-cancéreux de lignées de carcinome mammaire humain et les valeurs mesurées de STAFI et $K_{obs}$

La figure 3 représente la détermination de la valeur de delta mA max (STAFI, correspondant à la quantité d'actine polymérisée à l'état stationnaire) dans deux lignées cellulaires de mélanomes parentales, ayant un potentiel plus ou moins métastatique (B16F0 < B16F10). Le tableau 4 ci-dessous rapporte la cinétique de polymérisation de l'actine en présence d'extraits cellulaires par polarisation statique de fluorescence.

Tableau 4

| Lignées cellulaires | Delta mA max | Kobs |
|---|---|---|
| B16F0 | 47 | 0,071 |
| B16F10 | 37 | 0,028 |

La quantité d'actine polymérisée à l'état stationnaire (« STAFI » : valeur d'anisotropie de fluorescence au plateau, c'est-à-dire la valeur de delta mA maximum) de la lignée MCF7-Dox, résistante à la doxorubicine, est comparée à celle de la lignée parentale sensible (MCF7) (figure 3). Pour la lignée résistante (MCF7-Dox), le delta mA max est nettement inférieur au delta mA max de la lignée sensible (MCF7), soit 52mA et 71mA respectivement.

De plus, la valeur de delta mA max de la lignée résistante MCF7-MDR, transfectées par le gène codant pour la P-gp, est également comparée à la valeur de la lignée sensible (MCF7). Pour la lignée résistante (MCF7-MDR), la delta mA max est également très nettement inférieur au delta mA max de la lignée sensible (MCF7), soit 35 et 71 respectivement.

4) Corrélation entre le potentiel métastatique de lignées cellulaires de mélanome murin (B16F0 et B16F10) et les valeurs mesurées de STAFI et $K_{obs}$

La figure 4 représente la détermination de la valeur de delta mA max (STAFI, correspondant à la quantité d'actine polymérisée à l'état stationnaire) dans différentes lignées cellulaires adhérentes, sensibles (MCF7) et résistantes (MCF7 Dox et MCF7 MDR) à un traitement anticancéreux. Le tableau 5 ci-dessous rapporte la cinétique de polymérisation de l'actine en présence d'extraits cellulaires par polarisation statique de fluorescence.

Tableau 5

| Lignées cellulaires | Delta mA-max | Kobs |
|---|---|---|
| MCF7 | 71 | 0,0034 |
| MCF7-Doxorubicine | 52 | 0,0034 |
| MCF7 MDR | 35 | 0,071 |

La quantité d'actine polymérisée à l'état stationnaire (« STAFI » : valeur d'anisotropie de fluorescence au plateau, c'est-à-dire la valeur de delta mA maximum) de la lignée B16F10, sélectionnée pour son potentiel métastatique à partir de la lignée B16F0 (Nakamura, Yoshikawa et al. 2002), est comparée à celle de la lignée parentale moins métastatique (B16F0) (figure 4). Pour la lignée la plus agressive (B16F10), le delta mA max est nettement inférieur au delta mA max de la lignée non tumorigène (B16F0), soit 37 et 47 respectivement.

La même observation est faite pour la vitesse de polymérisation de l'actine, représentée par la constante $K_{obs}$. Pour la lignée la plus agressive (B16F10), $K_{obs}$ est nettement inférieur au $K_{obs}$ de la lignée la moins agressive (B16F0), soit 0,028 et 0,071 respectivement.

5) Identification de la jasplakinolide comme molécule restaurant le potentiel de polymérisation de l'actine à l'état stationnaire.

La figure 5 représente la restauration de la valeur de delta mA max (STAFI, correspondant à la quantité d'actine polymérisée à l'état stationnaire) de cellules tumorigènes (NIH 3T3 EF, notées EF) au niveau de la valeur de cellules non tumorigènes (NIH 3T3)par l'ajout de la jasplakinolide (notée jaspla)

La quantité d'actine polymérisée à l'état stationnaire (« STAFI » : valeur d'anisotropie de fluorescence au plateau, c'est-à-dire la valeur de delta mA maximum) de cellules tumorigènes (NIH 3T3 EF, notée EF) a été mesurée après ajout de 10μM de jasplakinolide dans le milieu de polymérisation. Le delta mA max des cellules tumorales déterminé à une valeur initiale de 35mA est restauré par l'ajout de jasplakinolide à une valeur proche de celle de la lignée non tumorigène (NIH 3T3), 66mA et 65 mA respectivement.

La même observation est faite pour la vitesse de polymérisation de l'actine, représentée par la constante. La jasplakinolide restaure le $K_{obs}$ d'une valeur égale à 0,04 vers une valeur qui est proche de la valeur obtenue avec les cellules non tumorigènes, soit 0,15 et 0,086 respectivement.

RÉFÉRENCES BIBLIOGRAPHIQUES

1) Damiano, J. S., L. A. Hazlehurst, et al. (2001). "Cell adhesion-mediated drug resistance (CAM-DR) protects the K562 chronic myelogenous leukemia cell line from apoptos is induced by BCR/ABL inhibition, cytotoxic drugs, and gamma-irradiation." Leukemia 15(8): 1232-9.
2) dit Faute, M. A., L. Laurent, et al. (2002). "Distinctive alterations of invasiveness, drug résistance and cell-cell organization in 3D-cultures of MCF-7, a human breast cancer cell line, and its multidrug resistant variant." Clin Exp Metastasis 19(2): 161-8.

3) Dugray, A., J.F., Geay, et al. (2001). "Rapid generation of a tetracycline-inducible BCR-ABL defective retrovirus using a single autoregulatory retroviral cassette." Leukemia 15: 1658-62.

4) Fradelizi, J., V. Noireaux, et al. (2001). "ActA and human zyxin harbour Arp2/3-independent actin-polymerization activity." Nat Cell Biol 3(8): 699-707.

5) Lopes, E. C., G. Ernst, et al. (2002). "Dissimilar invasive and metastatic behavior of vincristine and doxorubicin-resistant cell lines derived from a murine T cell lymphoid leukemia." Clin Exp Metastasis 19 (4): 283-90.

6) Machesky, L. M., R. D. Mullins, et al. (1999). "Scar, a WASp-related protein, activates nucleation of actin filaments by the Arp2/3 complex." Proc Natl Acad Sci U S A 96(7): 3739-44.

7) Mitsumoto, M., T. Kamura, et al. (1998). "Emergence of higher levels of invasive and metastatic properties in the drug resistant cancer cell lines after the repeated administration of cisplatin in tumor-bearing mice." J Cancer Res Clin Oncol 124 (11) : 607-14.

8) Nakamura, K., N. Yoshikawa, et al. (2002). "Characterization of mouse melanoma cell lines by their mortal malignancy using an experimental metastatic model." Life Sci 70(7): 791-8.

**Revendications**

1. Méthode d'analyse de l'agressivité tumorale de cellules cancéreuses comprenant la mesure de la quantité d'actine polymérisée à l'état stationnaire dans un lysat desdites cellules.

2. Méthode selon la revendication 1, **caractérisée en ce que** la mesure réalisée sur lysat est comparée à une ou plusieurs valeurs de référence de la quantité d'actine polymérisée à l'état stationnaire soit dans des cellules en culture spécifiques d'un phénotype soit dans des tissus provenant de prélèvements biologiques.

3. Méthode selon l'une des revendications 1 ou 2, **caractérisée en ce que** la quantité d'actine polymérisée correspond à la somme de toute l'actine sous forme F.

4. Méthode selon l'une des revendications 1 à 3, **caractérisée en ce que** la mesure de la quantité d'actine à l'état stationnaire est réalisée par polarisation statique de fluorescence en présence de monomères d'actine liés à un fluorochrome, lesquels sont incorporés dans les filaments d'actine (actine F) formés au cours de la polymérisation de l'actine endogène du lysat.

5. Méthode selon la revendication 4, **caractérisée en ce que** les monomères d'actine liés à un fluorochrome sont ajoutés au lysat cellulaire dans un rapport compris entre 1/80e et 1/1600e par rapport à la quantité d'actine endogène.

6. Méthode selon l'une quelconque des revendications précédentes, **caractérisée en ce qu'**elle comprend :

    - la lyse des cellules cancéreuses dans des conditions non-dénaturantes pour les protéines et l'élimination des débris cellulaires,
    - le dosage des protéines totales du lysat,
    - l'ajout de monomères d'actine liés à un fluorochrome,
    - l'ajout des substances nécessaires à la polymérisation de l'actine endogène et à la protection des protéines du lysat,
    - la mesure de la quantité d'actine polymérisée à l'état stationnaire dans le lysat.

7. Méthode d'identification de molécules susceptibles de présenter une activité anti-cancéreuse, **caractérisée en ce qu'**elle comprend la mise en oeuvre d'une méthode selon l'une quelconque des revendications 1 à 6 en présence d'une quantité adéquate de ladite molécule, et **en ce que** l'on détermine la capacité de ladite substance à restaurer une quantité d'actine polymérisée à l'état stationnaire correspondant à celle de cellules non agressives.

8. Application de la méthode selon l'une quelconque des revendications 1 6, à l'évaluation du caractère invasif desdites cellules.

9. Application de la méthode selon l'une quelconque des revendications 1 6, à l'évaluation de la tumorigénicité desdites cellules.

10. Application de la méthode selon l'une quelconque des revendications 1 6, à la prédiction de la sensibilité desdites cellules à un traitement anti-cancéreux.

11. Application selon la revendication 10, **caractérisé**E en ce que ledit traitement anti-cancéreux consiste en une radiothérapie ou une chimiothérapie.

12. Un kit pour la mise en oeuvre d'une méthode selon l'une quelconque des revendications 1 à 7, **caractérisé en ce qu'**il comprend :

    - un milieu de re-suspension des cellules pour la lyse des cellules,
    - les substances nécessaires à la polymérisation de l'actine endogène et à la protection des protéines du lysat,
    - une solution de monomères d'actine liés à un fluorochrome,
- un tampon de polymérisation de l'actine,
- un tampon général d'actine,
- éventuellement des extraits de référence de cellules agressives et non agressives.

**Claims**

1. Method for analysing the tumour aggressivity of cancer cells comprising the measurement of the quantity of polymerised actin at steady-state in a lysate of said cells.

2. Method of claim 1, **characterised in that** the measurement carried out in a lysate is compared to one or more reference values of the quantity of polymerised actin at steady-state either in specific cells in culture of a phenotype or in tissues taken from biological sampling.

3. Method according to any one of claims 1 or 2, **characterised in that** the quantity of polymerised actin corresponds to the sum of all of the F-form actin.

4. Method according to any one of claims 1 to 3, **characterised in that** the measurement of the quantity of actin at steady-state is carried out by static fluorescence polarisation in the presence of actin monomers bound to a fluorochrome, which are incorporated into actin filaments (F-actin) formed during the polymerisation of the endogenous actin of the lysate.

5. Method according to claim 4, **characterised in that** the actin monomers bound to a fluorochrome are added to the cellular lysate in a ratio ranging between 1/80th and 1/1600th in relation to the quantity of endogenous actin.

6. Method according to any one of the preceding claims, **characterised in that** it comprises:

   - the lysis of the cancer cells under non-denaturing conditions for the proteins and the elimination of cell debris,
   - the assay of the total quantity of lysate proteins,
   - the addition of actin monomers bound to a fluorochrome,
   - the addition of substances necessary for the polymerisation of the endogenous actin and for the protection of the lysate proteins,
   - the measurement of the quantity of polymerised actin at steady-state in the lysate.

7. Method of identifying molecules likely to have an anti-cancer activity, **characterised in that** it comprises the implementation of a method according to any one of claims 1 to 6, in the presence of an appropriate quantity of said molecule, and the determination of the capacity of said substance to restore a quantity of polymerised actin at steady-state corresponding to that of non-aggressive cells.

8. Application of the method according to any one of claims 1 to 6 to the evaluation of the invasive character of said cells.

9. Application of the method according to any one of claims 1 to 6 to the evaluation of the oncogenicity of said cells.

10. Application of the method according to any one of claims 1 to 6 to the prediction of the sensitivity of said cells to an anti-cancer treatment.

11. Application according to claim 10, **characterised in that** said anti-cancer treatment consists of radiotherapy or chemotherapy.

12. A kit for implementing a method as claimed in any of claims 1 to 7, **characterised in that** it comprises:

   - a cell re-suspension medium for the cell lysis,
   - the substances necessary for the polymerisation of the endogenous actin and for the protection of the lysate proteins,
   - a solution of actin monomers bound to a fluorochrome,
   - an actin polymerisation buffer,
   - a general actin buffer,
   - possibly reference aggressive and non-aggressive cell extracts.

**Patentansprüche**

1. Methode zur Analyse der tumorösen Aggressivität von Krebszellen, das Messen der Menge polymerisierten Actins in stationärem Zustand in einem Lysat der besagten Zellen umfassend.

2. Methode nach Anspruch 1, **dadurch gekennzeichnet, dass** die auf Lysat durchgeführte Messung mit einem oder mehreren Referenzwerten der Menge polymerisierten Actins in stationärem Zustand entweder in Zellen in Spezialkultur eines Phänotyps oder in Geweben aus biologischen Proben verglichen wird.

3. Methode nach einem der Ansprüche 1 oder 2, **dadurch gekennzeichnet, dass** die Menge polymerisierten Actins der Summe des ganzen Actins in F-Form entspricht.

4. Methode nach einem der Ansprüche 1 bis 3, **dadurch gekennzeichnet, dass** die Messung der Actinmenge in stationärem Zustand durch statische Fluoreszenzpolarisation in Anwesenheit von mit einem Fluorochrom verbundenen Actinmonomeren durchgeführt wird, die in die Actinfilamente (Actin F) inkorporiert sind, die im Laufe der Polymerisation des endogenen Actins des Lysats gebildet wurden.

5. Methode nach Anspruch 4, **dadurch gekennzeichnet, dass** die mit einem Fluorochrom verbundenen Actinmonomere dem Zelllysat in einem Verhältnis von 1/80$^e$ bis 1/1600$^e$ im Verhältnis zu der Menge des endogenen Actins hinzugefügt werden.

6. Methode nach einem der vorangehenden Ansprüche, **dadurch gekennzeichnet, dass** sie umfasst:

   - die Lyse der Krebszellen unter nicht denaturierenden Bedingungen für die Proteine und die Entfernung des Zellschrotts,
   - die Dosierung der gesamten Proteine des Lysats,
   - das Hinzufügen von mit einem Fluorochrom verbundenen Actinmonomeren,
   - das Hinzufügen der zur Polymerisation des endogenen Actins und zum Schutz der Proteine des Lysats notwendigen Substanzen,
   - das Messen der Menge polymerisierten Actins in stationärem Zustand in dem Lysat.

7. Methode zur Identifizierung von Molekülen, die eine antikanzerogene Wirkung aufweisen könnten, **dadurch gekennzeichnet, dass** sie die Durchführung einer Methode nach einem der Ansprüche 1 bis 6 in Anwesenheit einer adäquaten Menge des besagten Moleküls umfasst, und **dadurch**, dass die Fähigkeit der besagten Substanz zur Wiederherstellung einer Menge polymerisierten Actins in stationärem Zustand der Fähigkeit nicht aggressiver Zellen entspricht.

8. Anwendung der Methode nach einem der Ansprüche 1 bis 6 zur Beurteilung des invasiven Charakters der besagten Zellen.

9. Anwendung der Methode nach einem der Ansprüche 1 bis 6 zur Beurteilung der Tumorgenizität der besagten Zellen.

10. Anwendung der Methode nach einem der Ansprüche 1 bis 6 zur Voraussage der Sensibilität der besagten Zellen bei einer Anti-Krebs-Therapie.

11. Anwendung nach Anspruch 10, **dadurch gekennzeichnet, dass** die besagte Anti-Krebs-Therapie in einer Strahlentherapie oder einer Chemotherapie besteht.

12. Ein Set zur Durchführung einer Methode nach einem der Ansprüche 1 bis 7, **dadurch gekennzeichnet, dass** es umfasst:

   - ein Medium zur Resuspension der Zellen für die Lyse der Zellen,
   - die zur Polymerisation des endogenen Actins und zum Schutz der Proteine des Lysats notwendigen Substanzen,
   - eine Lösung von an ein Fluorochrom gebundenen Actinmonomeren,
   - einen Actin-Polymerisationspuffer,
   - ein allgemeiner Actin-Puffer,
   - eventuell Referenzextrakte aggressiver und nicht aggressiver Zellen.

## Figure 1

**Temps d'incubation (sec)**

- ■ NIH 3T3
- ▲ NIH 3T3 EF
- ⊕ NIH 3T3 EF zyxine

## Figure 2

**Temps d'incubation (sec.)**

- ■ BAF3 parentales
- ▲ BAF3 Bcr-Abl +
- ▼ BAF3 Bcr-Abl -

Figure 3

**Temps d'incubation (sec)**

Figure 4

**Temps d'incubation (sec)**

Figure 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **AMIANO, J. S ; L. A. HAZLEHURST et al.** Cell adhesion-mediated drug resistance (CAM-DR) protects the K562 chronic myelogenous leukemia cell line from apoptos is induced by BCR/ABL inhibition, cytotoxic drugs, and gamma-irradiation. *Leukemia,* 2001, vol. 15 (8), 1232-9
- **FAUTE, M. A ; L. LAURENT et al.** Distinctive alterations of invasiveness, drug résistance and cell-cell organization in 3D-cultures of MCF-7, a human breast cancer cell line, and its multidrug resistant variant. *Clin Exp Metastasis,* 2002, vol. 19 (2), 161-8
- **DUGRAY, A. ; J.F., GEAY et al.** Rapid generation of a tetracycline-inducible BCR-ABL defective retrovirus using a single autoregulatory retroviral cassette. *Leukemia,* 2001, vol. 15, 1658-62
- **FRADELIZI, J ; V. NOIREAUX et al.** ActA and human zyxin harbour Arp2/3-independent actin-polymerization activity. *Nat Cell Biol,* 2001, vol. 3 (8), 699-707
- **LOPES, E. C ; G. ERNST et al.** Dissimilar invasive and metastatic behavior of vincristine and doxorubicin-resistant cell lines derived from a murine T cell lymphoid leukemia. *Clin Exp Metastasis,* 2002, vol. 19 (4), 283-90
- **MACHESKY, L. M ; R. D. MULLINS et al.** Scar, a WASp-related protein, activates nucleation of actin filaments by the Arp2/3 complex. *Proc Natl Acad Sci U S A,* 1999, vol. 96 (7), 3739-44
- **MITSUMOTO, M ; T. KAMURA et al.** Emergence of higher levels of invasive and metastatic properties in the drug resistant cancer cell lines after the repeated administration of cisplatin in tumor-bearing mice. *J Cancer Res Clin Oncol,* 1998, vol. 124 (11), 607-14
- **NAKAMURA, K ; N. YOSHIKAWA et al.** Characterization of mouse melanoma cell lines by their mortal malignancy using an experimental metastatic model. *Life Sci,* 2002, vol. 70 (7), 791-8